# EUROPEAN PATENT APPLICATION

(11) **EP 3 138 473 A1**
(43) Date of publication of application: **08.03.2017**
(21) Application number: 16186746.0
(22) Date of filing: 01.09.2016
(51) Int. Cl.: A61B 3/10, A61B 3/14

(54) **SCANNING LASER OPHTHALMOSCOPE**

(30) Priority: 02.09.2015 JP 2015172942
(71) Applicant: NIDEK CO., LTD, Gamagori-shi Aichi 443-0038 (JP)
(72) Inventor: SASAKI, Joji, Gamagori-shi, Aichi 443-0038 (JP); HANEBUCHI, Masaaki, Gamagori-shi, Aichi 443-0038 (JP)
(74) Representative: Zimmermann, Tankred Klaus

(57) **Abstract**

Provided is a scanning laser ophthalmoscope (1) including: a light projecting optical system (10), including a light source (12), for projecting a line-shaped light flux to an examinee's eye via an objective optical system (45); a light receiving optical system (20), including an optical detector (22), for receiving light from the examinee's eye resulting from the line-shaped light flux via the objective optical system; an optical scanner (40) for scanning the examinee's eye with the line-shaped light flux; and a focus adjustment unit including a drive mechanism (50) for moving the light source (12) and the optical detector (22) relative to the optical scanner (40) and the objective optical system (45).

## Description

### CROSS-REFERENCE TO RELATED APPLICATION

This application claims priority from Japanese Patent Application No. 2015-172942 filed with the Japan Patent Office on September 2, 2015, the entire contents of which are hereby incorporated by reference.

### BACKGROUND

### 1. Technical Field

The present disclosure relates to a scanning laser ophthalmoscope.

### 2. Related Art

A line scan SLO (SLO: Scanning Laser Ophthalmoscope) is known as a scanning laser ophthalmoscope that scans an examinee's eye to obtain an image of the examinee's eye. According to the line scan SLO, the fundus is scanned with a line-shaped (linear) light flux; accordingly, an SLO image of the fundus can be obtained. For example, line scan SLOs disclosed in JP-T-2005-529669 and JP-A-08-206079 are known as typical line scan SLOs.

According to JP-T-2005-529669, when the diopter is corrected, an objective lens moves. On the other hand, according to JP-A-08-206079, when the diopter is corrected, lenses placed respectively in a light projecting system and a light receiving system move.

### SUMMARY

A scanning laser ophthalmoscope according to an embodiment of the present disclosure includes: a light projecting optical system, including a light source, for projecting a line-shaped light flux to an examinee's eye via an objective optical system; a light receiving optical system, including an optical detector, for receiving light from the examinee's eye resulting from the line-shaped light flux via the objective optical system; an optical scanner for scanning the examinee's eye with the line-shaped light flux; and a focus adjustment unit including a drive mechanism for moving the light source and the optical detector relative to the optical scanner and the objective optical system.

### BRIEF DESCRIPTION OF DRAWINGS

Fig. 1 is a diagram illustrating an example of an optical system of an apparatus according to the embodiment;
Fig. 2 is a diagram illustrating an example of a control system of the apparatus according to the embodiment.

### DETAILED DESCRIPTION

In the following detailed description, for purpose of explanation, numerous specific details are set forth in order to provide a thorough understanding of the disclosed embodiments. It will be apparent, however, that one or more embodiments may be practiced without these specific details. In other instances, well-known structures and devices are schematically shown in order to simplify the drawing.

Line scan SLOs such as above are susceptible to improvement in, for example, light projecting and receiving systems, a focus system, and focus control. For example, according to JP-T-2005-529669, the distance between an examinee's eye and the objective lens changes in the diopter correction. In this case, the relative position between the examinee's eye and the apparatus may be readjusted. On the other hand, according to JP-A-08-206079, control in the diopter correction is relatively difficult.

The technical problem of the present disclosure is to provide a scanning laser ophthalmoscope in which at least one of the known technologies has been improved.

In order to solve the above problem, the scanning laser ophthalmoscope of the present disclosure includes the following configuration.

A scanning laser ophthalmoscope according to the embodiment includes: a light projecting optical system, including a light source, for projecting a line-shaped light flux to an examinee's eye via an objective optical system; a light receiving optical system, including an optical detector, for receiving light from the examinee's eye resulting from the line-shaped light flux via the objective optical system; an optical scanner for scanning the examinee's eye with the line-shaped light flux; and a focus adjustment unit including a drive mechanism for moving the light source and the optical detector relative to the optical scanner and the objective optical system.

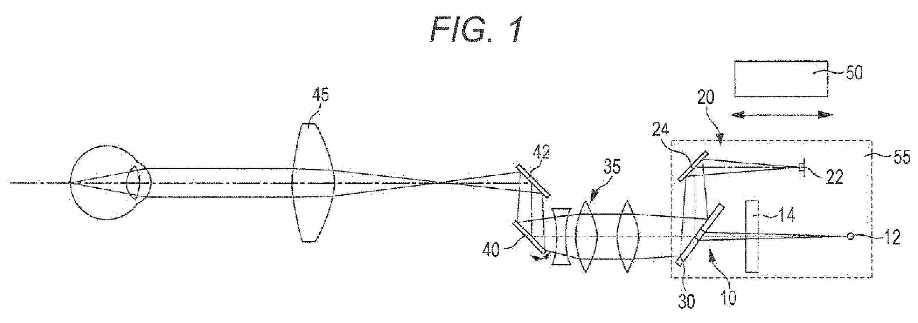

For example, a line scanning ophthalmoscope (line scan SLO) may be used as a scanning laser ophthalmoscope (hereinafter, SLO apparatus) 1 of the embodiment (see Fig. 1). The line scan SLO is, for example, an apparatus that scans a line-shaped light flux to obtain a confocal image of an examinee's eye. However, the SLO apparatus of the embodiment may include an optical system having a confocal point in a lateral direction of the line-shaped light flux. A description is given hereinafter taking imaging of the fundus of the examinee's eye as an example. However, the embodiment can also be applied to an apparatus configured to be capable of imaging the anterior segment.

The line scanning ophthalmoscope according to the embodiment may include, for example, a light projecting optical system 10, a light receiving optical system 20, an optical path separator 30, an image forming lens 35, an optical scanner 40, an objective lens 45, a drive mechanism (actuator) 50, and a calculation controller (calculation control unit) 70.

The light projecting optical system 10 may, for example, be configured to project a line-shaped light flux to the fundus via the objective lens 45. The light projecting optical system 10 may include, for example, an SLO light source 12 and a cylindrical lens 14.

The SLO light source 12 may be, for example, a laser light source or an SLD (superluminescent diode) light source. The shape of the light source 12 may be point-like. The SLO light source 12 may be placed at a position conjugate with the fundus of the examinee's eye. The SLO light source 12 may be a light source that emits at least one of infrared light and visible light. The use of a light source that emits infrared light as the SLO light source 12 is advantageous in photography in a non-mydriatic state. Moreover, a white light source may be used as the SLO light source 12. The white light source is advantageous in color photography.

The cylindrical lens 14 may be configured to converge the light from the SLO light source 12 in a one-dimensional direction. The cylindrical lens 14 may, for example, be configured to have refractive power in a direction perpendicular to a scan direction of the optical scanner 40 and not have refractive power in the scan direction of the optical scanner 40. The cylindrical lens 14 may be configured to converge the light from the SLD light source 12 in the direction perpendicular to the scan direction. The light from the SLD light source 12 is diverged in the direction without refractive power. Naturally, the above configuration can be modified according to the configuration of the entire optical system.

The light receiving optical system 20 may, for example, be configured to receive light from the examinee's eye resulting from the line-shaped light flux via the objective lens 45. The light receiving optical system 20 may include, for example, an optical detector 22. The optical detector 22 may be, for example, a one-dimensional photo detector (one-dimensional camera). However, a one-dimensional CCD sensor, one-dimensional CMOS sensor, or the like may be used. The optical detector 22 may be placed at a position conjugate with the fundus of the examinee's eye. In the embodiment, light from the optical path separator 30 is received by the optical detector 22 via a planar mirror 24. Naturally, the light from the optical path separator 30 can also enter the optical detector 22 directly, instead of providing the planar mirror 24.

The optical path separator 30 may, for example, be configured to separate an optical path of the light projecting optical system 10 and an optical path of the light receiving optical system 20. The optical path separator 30 may be placed in such a manner as that both an optical axis of the light projecting optical system 10 and an optical axis of the light receiving optical system become coaxial downstream of the optical path separator 30. In Fig. 1, the SLO light source 12 and the optical detector 22 are placed on an extension of an optical axis of the image forming lens 35 via the optical path separator 30.

The optical path separator 30 may have, for example, the characteristic of reflecting one of the light from the SLO light source 12 and the light from the examinee's eye and transmitting the other (including passing the light through). The optical path separator 30 may include, for example, a light reflection part and a light transmission part. In that case, the optical path separator 30 may be configured in such a manner as that one of the light reflection part and the light transmission part is formed in a central portion and the other is formed in its surrounding portion. In this case, the central portion may transmit one of the light from the SLO light source 12 and the light from the examinee's eye and the surrounding portion may reflect the other. Moreover, the central portion may reflect one of the light from the SLO light source 12 and the light from the examinee's eye and the surrounding portion may transmit the other.

In Fig. 1, the light from the SLO light source 12, which the optical path separator 30 has allowed to transmit, is guided toward the examinee's eye. In addition, the light from the examinee's eye is reflected by the optical path separator 30 to be guided toward the optical detector 22. In this case, the reflected light from the cornea and the lens system proceeds toward the SLO light source 12. Hence, noise light to the optical detector 22 is reduced.

The optical path separator 30 may be, for example, a hole mirror. The hole mirror opens at a central portion and includes a mirror surface in its surrounding portion. The optical path separator 30 is not limited to the hole mirror. The optical path separator 30 may be configured in such a manner as that a surrounding portion of a light transmitting plate (for example, a glass plate) is coated with a mirror surface. The optical path separator 30 may be a half-mirror.

The image forming lens 35 may, for example, be configured to temporarily form an image of the light from the SLO light source 12 at a front focus position of the objective lens 45 and also form an image of the light from the examinee's eye on the optical detector 22. The image forming lens 35 may include, for example, a group of lenses. The image forming lens 35 may be placed upstream of the optical scanner 40. The image forming lens 35 may be placed on a common optical path of the light projecting optical system 10 and the light receiving optical system 20. The image forming lens 35 may be placed between the optical scanner 40 and the optical path separator 30. In the embodiment, assume that the SLO light source 12 side is upstream and the examinee's eye side is downstream with respect to the light projecting optical system 10, and the optical detector 22 side is upstream and the examinee's eye side is downstream with respect to the light receiving optical system 20.

The optical scanner 40 may, for example, be configured to scan the examinee's eye with a line-shaped light flux. The optical scanner 40 may scan the examinee's eye with the line-shaped light flux in a predetermined direction. The scan direction may be, for example, the vertical direction or the horizontal direction.

The optical scanner 40 may be, for example, a galvanometer mirror. The angle of reflection of the mirror is freely adjusted by the drive mechanism. A direction of reflection (progression) of the light emitted from the SLO light source 12 is changed by the optical scanner 40 and also is used for a scan on the examinee's eye. Examples of the usable optical scanner 40 include an acousto-optic modulator (AOM) that changes the direction of progression (deflection) of light in addition to reflection mirrors (a galvanometer mirror, a polygon mirror, and a resonant scanner).

The optical scanner 40 may be placed on the common optical path of the light projecting optical system 10 and the light receiving optical system 20. In this case, the optical scanner 40 scans with the light from the SLO light source 12 and also descans with the light from the examinee's eye. The optical scanner 40 may be placed, for example, between the objective lens 45 and the optical path separator 30. The optical scanner 40 may be placed at a position conjugate with the pupil of the examinee's eye.

The optical scanner 40 may be placed at a downstream focus position of the image forming lens 35. As a result, the optical path upstream of the optical scanner 40 forms a telecentric optical system. Consequently, the change of a photographing magnification can be suppressed in the diopter correction.

The objective lens 45 may be configured to guide the light from the optical scanner 40 to the examinee's eye (the fundus) and also return the reflected light from the examinee's eye to the optical scanner 40. The objective lens 45 may include a group of lenses including at least one objective lens. For example, a group of objective lenses may allow the optical scanner 40 to be placed at the position conjugate with the pupil of the examinee's eye (however, Fig. 1 illustrates only one lens). Consequently, the objective optical system is simply required to include only the group of lenses. In other words, the objective optical system does not need to include a plurality of lens groups. As a result, the configuration of the optical system can be simplified. In the embodiment, the group of lenses is defined as a single lens, or at least two lenses configured to function as a single lens by being placed at positions close to each other. The objective lens 45 may be placed on the common optical path of the light projecting optical system 10 and the light receiving optical system 20.

The drive mechanism 50 may, for example, be configured to adjust focus for the fundus of the examinee's eye upon photographing of the fundus. In this case, the drive mechanism 50 may, for example, be configured to correct the diopter of the examinee's eye.

The drive mechanism 50 may move, for example, an optical member group 55 including at least the SLO light source 12 and the optical detector 22 relative to the optical scanner 40 and the objective lens 45. The optical member group 55 is simply required to include, for example, at least the SLO light source 12 and the optical detector 22. The optical member group 55 may be an optical member group placed upstream of the optical path separator 30 (see Fig. 1).

The drive mechanism 50 may move the optical member group 55, for example, in a direction where the diopter of the examinee's eye is corrected. The drive mechanism 50 may be an actuator. The optical member group 55 may be moved by, for example, the drive of a motor.

The drive mechanism 50 may, for example, move the optical member group 55 collectively. As a result, the diopter correction using the light projecting and light receiving systems collectively becomes possible. Hence, highly accurate control for the diopter correction is not necessarily required. In this case, the drive mechanism 50 may collectively move the light projecting optical system 10 placed on the SLO light source 12 side with respect to the optical path separator 30, and the light receiving optical system 20 placed on the optical detector 22 side with respect to the optical path separator 30. For example, the drive mechanism 50 may move one stage where the optical member group 55 is placed.

The drive mechanism 50 may move the optical path separator 30 together with the optical member group 55 (see Fig. 1). Consequently, the possibility of misalignment of the optical axes of the light projecting optical system 10 and the light receiving optical system 20 can be reduced.

The drive mechanism 50 may move the optical member group 55 collectively relative to the objective lens 45, the optical scanner 40, and the image forming lens 35 (see Fig. 1). Consequently, highly accurate control for the diopter correction is not required. In addition, the moving portion can be relatively reduced in size. Moreover, as described above, even if a telecentric optical system is formed by the image forming lens 35 upstream of the optical scanner 40, the change of the photographing magnification of when the optical member group 55 is moved is suppressed.

The light (for example, laser light) emitted from the SLO light source 12 is collimated by the cylindrical lens 14 in a one-dimensional direction. The collimated light then passes through the optical path separator 30. The light that has passed through the optical path separator 30 is converged by the image forming lens 35. The direction of reflection of the converged light is then changed by the optical scanner 40. The light deflected by the optical scanner 40 is applied to the fundus of the examinee's eye via a mirror 42 and the objective lens 45.

In this case, the light from the optical scanner 40, which proceeds in the direction of a scan by the optical scanner 40, is temporarily condensed via the mirror 42. The condensed light is then condensed on the fundus of the examinee's eye via the objective lens 45. Moreover, the light from the cylindrical lens 14, which proceeds in the direction perpendicular to the scan direction of the optical scanner 40, is temporarily condensed by the image forming lens 35 on the optical scanner 40. The condensed light is then reflected by the mirror 42. The reflected light is temporarily condensed on the pupil via the objective lens 45. The condensed light is then applied to the fundus of the examinee's eye. As a result, a line-shaped (linear) light flux is projected onto the fundus of the examinee's eye. The fundus is scanned with the line-shaped light flux in the scan direction of the optical scanner 40.

The light reflected by the fundus is received by the optical detector 22 through the objective lens 45, the mirror 42, the optical scanner 40, the image forming lens 35, the optical path separator 30, and the planar mirror 24. According to the embodiment, the light from the optical path separator 30 is detected by the optical detector 22 not via the confocal opening. However, the optical detector 22 is placed at the position conjugate with the fundus to hold the confocal characteristics. Naturally, slit light may be placed in front of the optical detector 22.

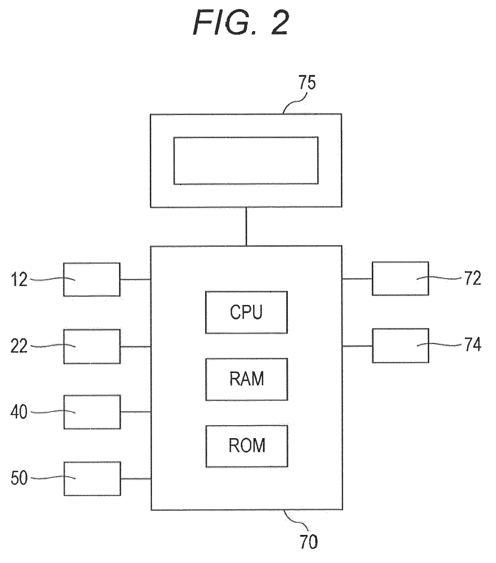

The light receiving signal detected by the optical detector 22 is input into the control unit 70. The control unit 70 (see Fig. 2) generates a front image (SLO image) of the examinee's eye based on the light receiving signal obtained by the optical detector 22. The generated front image is stored in a memory 72. The SLO image is acquired by a scan by the optical scanner 40. The front image may be generated based on the positional relationship between the scan position of the optical scanner 40 and the light receiving signal. The control unit 70 may be configured to repeatedly drive the optical scanner 40 and accordingly obtain a moving image of the front image.

The control unit 70 may include, for example, a CPU (processor), a RAM, and a ROM (see Fig. 2). For example, the CPU of the control unit 70 is responsible for the control of the SLO apparatus 1. Various kinds of information are temporarily stored in the RAM. For example, various programs for controlling the operation of the SLO apparatus 1 and initial values are stored in the ROM of the control unit 70.

The control unit 70 may be electrically connected to the memory 72, an operating unit 74, a display unit 75, and the like. A non-transitory storage medium that can hold the storage content even if the supply of power is stopped may be used as the memory 72. For example, a hard disk drive, a flash ROM, or a USB memory that is detachably installed on the SLO apparatus 1 may be used as the memory 72. A photographed SLO image may be stored in the memory 72. The control unit 70 may control the display unit 75. For example, the generated SLO image may be displayed on the display unit 75. Various operation instructions of an examiner may be input into the operating unit 74. The operating unit 74 may include a user interface (for example, a mouse, a touchscreen, or a joy stick) for accepting the examiner's instruction. Furthermore, the operating unit 74 may include, for example, a focus adjustment switch for the examinee's eye or a release switch for capturing an SLO image.

The operation of the scanning laser ophthalmoscope of the embodiment, which is configured as described above, is briefly described. The examiner aligns the photographing optical axis with the examinee's eye on a screen photographed by an unillustrated anterior segment camera. Next, the examiner asks the examinee to fixate a fixation light projected by an unillustrated fixation target projecting optical system. The examiner guides the examinee to a desired photographing portion in this manner. An SLO image of the fundus is displayed on the display unit 75. The examiner then brings a focus to the fundus based on the SLO image on the display unit 75 by means of the operating unit 74.

The control unit 70 controls the drive of the drive mechanism 50 based on an operation signal from the operating unit 74. For example, the control unit 70 moves the drive mechanism 50 in a plus direction or a minus direction (for example, a direction in which the optical member group 55 is brought close to the optical scanner 40 and the objective optical system or a direction in which the optical member group 55 moves away from them) of the examinee's eye according to the operation direction and the amount of operation, which are input from the operating unit 74. The plus direction is, for example, a direction in which the optical member group 55 moves closer to the optical scanner 40 and the objective optical system. The minus direction is a direction in which the optical member group 55 moves away from the optical scanner 40 and the objective optical system. The drive of the drive mechanism 50 allows at least the optical member group 55 to move. As a result, the focus level of the line-shaped light flux for the fundus and the optical detector 22 is adjusted. Accordingly, the focus level of the SLO image is adjusted so that the SLO image in focus can be obtained. The diopter correction is not limited by a manual operation. Autofocusing may be performed.

The diopter of the examinee's eye is corrected as described above. The examiner can subsequently adjust various photographing conditions, looking at a moving image of the SLO image displayed on the display unit 75. The examiner can capture the SLO image by means of the operating unit 74. In this case, the control unit 70 captures the SLO image based on an operation signal from the operating unit 74. The captured SLO image is stored in the memory 72.

As illustrated above, the optical member group including at least the light source and the optical detector moves relative to the optical scanner and the objective optical system (for example, the objective lens). In this case, in the diopter correction, the relative position between the objective optical system and the examinee's eye in a Z direction (a direction along the optical axis) is not necessarily required to be readjusted. Consequently, for example, the positional relationship between the examinee's eye and the apparatus can be maintained. Moreover, the moving unit of the diopter correction mechanism can be relatively small.

Moreover, the objective optical system includes a group of lenses. In addition, the image forming optical system (for example, the image forming lens) is placed upstream of the optical scanner. Furthermore, the optical member group placed upstream of the image forming optical system moves. Consequently, the objective optical system is simplified. In addition, the moving unit of the diopter correction mechanism can be relatively small.

Modifications of the embodiment are illustrated below. In the above description, the drive mechanism 50 moves the optical path separator 30 together with the optical member group 55. However, the drive mechanism used in the embodiment is not limited to the drive mechanism 50. For example, the optical path separator 30 may be fixed to the optical scanner 40 and the objective lens 45 in the diopter correction.

Moreover, not all the optical members placed upstream of the optical path separator 30, as the diopter correction mechanism, need to move. For example, even if the drive mechanism 50 moves only the SLO light source 12, the cylindrical lens 14, and the optical detector 22 in Fig. 1, certain effects can be obtained as the diopter correction. In other words, it is sufficient if the diopter correction is possible to some extent.

The above light projecting optical system includes a point-like light source as the SLO light source 12. Moreover, a line-shaped light flux is generated by means of the cylindrical lens 14. However, the optical member for generating a line-shaped light flux is not limited to this. For example, a line-like light source as the SLO light source 12 may generate a line-shaped light flux. In this case, the use of the cylindrical lens is not necessarily required. Point light sources arranged one-dimensionally, or a line emission type light source may be used as the line-like light source. Other examples include a light projecting optical system configured to include a slit opening at a position conjugate with the fundus and include a light source behind the slit opening. The slit opening is illuminated to form the line-like light source.

The above light projecting optical system may include a plurality of light sources having different emission wavelengths, as the SLO light source 12. For example, the light projecting optical system may include an infrared light source and a visible light source. In this case, light fluxes emitted from the light sources are combined by a dichroic mirror or the like.

The above light projecting optical system uses the objective lens 45 as the objective optical system and the image forming lens 35 as the image forming optical system. However, at least one of the objective optical system and the image forming optical system may use a mirror system (for example, a concave mirror). At least one of the objective optical system and the image forming optical system may be configured to be able to combine a lens system and a mirror system.

According to the above description, with respect to the optical path separator 30, the light projecting optical system 10 is placed in the transmission direction, and the light receiving optical system 20 is placed in the reflection direction. However, the arrangement is not limited to the above one. The light projecting optical system 10 may be placed in the reflection direction, and the light receiving optical system 20 may be placed in the transmission direction. The optical path separator 30 may include, for example, the light reflection part in the central portion and the light transmission part in the surrounding portion. Moreover, the optical path separator 30 may include, for example, the light reflection part in the surrounding portion and the light transmission part in the central portion. In this case, the optical arrangement of the light projecting optical system 10 and the light receiving optical system 20 can be designed in accordance with the configuration of the optical path separator 30, as appropriate.

The SLO apparatus 1 may be a composite apparatus with another apparatus. The composite apparatus may include, for example, an optical path separator (for example, a dichroic mirror or a half-mirror) between the objective lens 45 and the optical scanner 40. In this case, their optical axes become coaxial.

For example, the SLO apparatus 1 may include an OCT optical system (optical coherence tomography optical system). An SLO image may be used for positioning of OCT and the like. In this case, the relative position between the objective lens 45 and the examinee's eye is not necessarily required to be adjusted in the diopter correction. Hence, an SLO image and an OCT image can be smoothly taken. Moreover, the line scan SLO can increase the frame rate of when an SLO image is obtained. Hence, the speed of tracking of OCT light can be increased.

For example, the SLO apparatus 1 may include a therapeutic laser irradiation optical system. An SLO image may be used for the positioning of therapeutic laser. In this case, the relative position between the objective lens 45 and the examinee's eye is not necessarily required to be adjusted in the diopter correction. Hence, laser can be applied smoothly. Moreover, the line scan SLO can increase the frame rate of when an SLO image is obtained. Hence, the tracking speed of the therapeutic laser can be increased.

In the above description, the scanning laser ophthalmoscope configured to photograph the fundus is described as an example. However, the embodiment is not limited to this, and can also be applied to a scanning laser ophthalmoscope configured to photograph the anterior segment. In this case, the drive mechanism 50 may be configured to adjust focus for the anterior segment of the examinee's eye.

The scanning laser ophthalmoscope according to the embodiment of the present disclosure may be the following first to fifth scanning laser ophthalmoscopes.

The first scanning laser ophthalmoscope includes: a light projecting optical system, including an SLO light source, for projecting a line-shaped light flux to an examinee's eye via an objective optical system; a light receiving optical system, including an optical detector, for receiving light from the examinee's eye resulting from the line-shaped light flux via the objective optical system; an optical scanner for scanning the examinee's eye with the line-shaped light flux; and a focus adjustment unit including a drive mechanism for moving an optical member group at least including the light source and the optical detector relative to the optical scanner and the objective optical system.

The second scanning laser ophthalmoscope is the first scanning laser ophthalmoscope characterized in that the objective optical system includes a group of lenses including at least one objective lens, and the group of lenses allows the optical scanner to be placed at a position conjugate with the pupil of the examinee's eye.

The third scanning laser ophthalmoscope is the first or second scanning laser ophthalmoscope characterized in that an image forming optical system is placed upstream of the optical scanner on a common optical path of the light projecting optical system and the light receiving optical system, and the drive mechanism moves the optical member group relative to the optical scanner, the objective optical system, and the image forming optical system.

The fourth scanning laser ophthalmoscope is the third scanning laser ophthalmoscope characterized in that the scanner is placed at a downstream focus position of the image forming optical system, and an optical path upstream of the image forming optical system is formed as a telecentric optical system.

The fifth scanning laser ophthalmoscope is any one of the first to fourth scanning laser ophthalmoscopes characterized by an optical path separator for separating an optical path of the light projecting optical system and an optical path of the light receiving optical system. The drive mechanism moves the optical member group and the optical path separator relative to the optical scanner and the objective optical system.

The foregoing detailed description has been presented for the purposes of illustration and description. Many modifications and variations are possible in light of the above teaching. It is not intended to be exhaustive or to limit the subject matter described herein to the precise form disclosed. Although the subject matter has been described in language specific to structural features and/or methodological acts, it is to be understood that the subject matter defined in the appended claims is not necessarily limited to the specific features or acts described above. Rather, the specific features and acts described above are disclosed as example forms of implementing the claims appended hereto.

## Claims

1. A scanning laser ophthalmoscope comprising:
a light projecting optical system, including a light source, for projecting a line-shaped light flux to an examinee's eye via an objective optical system;
a light receiving optical system, including an optical detector, for receiving light from the examinee's eye resulting from the line-shaped light flux via the objective optical system;
an optical scanner for scanning the examinee's eye with the line-shaped light flux; and
a focus adjustment unit including a drive mechanism for moving the light source and the optical detector relative to the optical scanner and the objective optical system.

2. The scanning laser ophthalmoscope according to claim 1, wherein
the objective optical system includes a group of lenses including at least one objective lens.

3. The scanning laser ophthalmoscope according to claim 2, wherein
the group of lenses allows the optical scanner to be placed at a position conjugate with the pupil of the examinee's eye.

4. The scanning laser ophthalmoscope according to any one of claims 1 to 3, wherein
an image forming optical system is placed upstream of the optical scanner on a common optical path of the light projecting optical system and the light receiving optical system.

5. The scanning laser ophthalmoscope according to claim 4, wherein
the drive mechanism is configured to move the light source and the optical detector relative to the optical scanner, the objective optical system, and the image forming optical system.

6. The scanning laser ophthalmoscope according to claim 4 or 5, wherein
the optical scanner is placed at a downstream focus position of the image forming optical system.

7. The scanning laser ophthalmoscope according to any one of claims 4 to 6, wherein
an optical path upstream of the image forming optical system is configured to form a telecentric optical system.

8. The scanning laser ophthalmoscope according to any one of claims 1 to 7, comprising an optical path separator for separating an optical path included in the light projecting optical system and an optical path included in the light receiving optical system, wherein the drive mechanism is configured to move the light source, the optical detector, and the optical path separator relative to the optical scanner and the objective optical system.

9. The scanning laser ophthalmoscope according to any one of claims 1 to 8, further comprising an optical coherence tomography optical system.

10. The scanning laser ophthalmoscope according to any one of claims 1 to 9, further comprising a therapeutic laser irradiation optical system.

11. The scanning laser ophthalmoscope according to any one of claims 1 to 10, configured to photograph fundus.

12. The scanning laser ophthalmoscope according to any one of claims 1 to 11, wherein
the drive mechanism is configured to adjust focus for the fundus.
